# EUROPEAN PATENT APPLICATION

(11) **EP 4 768 465 A1**
(43) Date of publication of application: **01.07.2026**
(21) Application number: 24307320.2
(22) Date of filing: 27.12.2024
(51) Int. Cl.: C07C 47/21, C07C 47/225, C07C 47/277, C07C 69/52, C07C 69/602, C07C 69/608, A23L 27/20

(54) **FLORAL CHEMICAL COMPOUNDS AS SUBSTITUTES FOR 3-(4-TERT-BUTYLPHENYL)-2-METHYLPROPANAL**

(71) Applicant: V. Mane Fils, 06620 Le Bar-sur-Loup (FR)
(72) Inventor: MURATORE, Agnès, 06740 Châteauneuf-Grasse (FR); TECI, Matthieu, 67118 Geispolsheim (FR)
(74) Representative: Murgitroyd & Company

(57) **Abstract**

A compound which may be for use in perfumery or organoleptic applications is provided, where the compound has a general Formula (I): 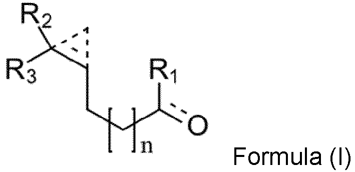 wherein n = 1 or 2;
wherein R¹ = H, methyl, ethyl, or alkoxy;
wherein R² = H, methyl, or ethyl; and R³ = ethyl, 2,4-dimethylcyclohex-3-enyl, 1-ethylpropyl, 1,5-dimethylhex-4-enyl, cyclopentyl, 1,5-dimethylhexyl, 2,6-dimethylhepta-1,5-dienyl, cyclohexyl, 3,3-dimethylcyclohexyl, 2,6-dimethylhept-5-enyl, 2-phenylpropyl, 4-methylpent-3-enyl, hex-3-enyl, 1,5-dimethyl-5-methoxyhexyl, or 1-methylpropyl; or
wherein R² and R³ taken together form 4-propylcyclohexyl, 3,3-dimethylcyclohexyl, or 4-methylcyclohexyl. Methods of making and using same are further provided.

## Description

### FIELD OF THE INVENTION

The present invention relates generally to floral chemical compounds, and more particularly to floral chemical compounds suitable as substitutes for 3-(4-tert-Butylphenyl)-2-methylpropanal.

### BACKGROUND OF THE INVENTION

Compounds having muguet odor characteristics are highly used as perfume ingredients. These compounds are important ingredients in floral bases and can act as harmonizers across many different types of fragrance creations. Compounds of this type are used widely in consumer products, such as personal care and consumer care products, as well as in fine perfumery, to generate pleasant odors or to mask unpleasant odors.

A perfume ingredient widely valued for its muguet odor note is 3-(4-tert-butylphenyl)-2-methylpropanal (CAS 80-54-6), which is commonly known under the tradename LILIAL^{™}. Previously, this compound had found wide use in fine perfumery, as well as in personal and household care products. However, recently its use has come under regulatory scrutiny in view of findings that it exhibits toxic effects on the reproductive organs of some animals. As such, under the Global Harmonized System (GHS) classification system this compound was classified as a CMR2 material and in 2002, LILIAL^{™} was banned for use in cosmetics in the European Union. In view of the regulatory situation of Lilial^{™}, there is a need to replace it with other perfume ingredients.

In this regard, WO2010/105874A1 discloses compositions that can be used as a LILIAL^{®} surrogate. While the amount of LILIAL^{®} in the composition is reduced, some still remains in the formulation. Thus, at best, WO2010/105874A1 teaches how to limit LILIAL^{®} to a lesser amount in such composition, but not how to remove or replace it. And WO2010/105873A2 discloses compositions comprising oxazolidine derivatives, in combination with one or more other compounds, that could be used to substitute for LILIAL^{®}. Compounds that are mentioned include burgeonal, dimethylphenylethylcarbinol, MAYOL^{®}, mugetanol, citrusal, silvial, cyclamenaldehyde and FLOROL^{®}. The compositions described in this document require the presence of certain compounds such as burgeonal, silvial, and cyclamenaldehyde which are also starting to face raising questions on a toxicologal level. Thus, the document does not present a practical or long-term solution to the replacement of LILIAL^{®}.

Likewise, WO2009027957 proposes a solution residing in the formulation of combinations of known perfume ingredients from the perfumery palette.

WO2013045301 also proposes a solution to Lilial^{™} replacement, using two primary ingredients, namely (2,5-dimethyl-2,3-dihydro-1H-inden-2-yl) methanol (also known as Lilyflore^{™} (Firmenich SA)) and 3-(gem-dimethyl-5-indanyl)propanal, (also known as HIVERNAL^{®} (Firmenich SA)), in combination with other secondary perfuming ingredients.

WO2021075517A1 describes a new methylbenzene-containing compound used in fragrance composition and as substitute fragrance for Lilial^{™}.

Accordingly, there remains a need for new compounds that are useful as a LILIAL^{®} replacements in standard perfuming formulations that provide floral (lily-of-the-valley) odor characteristics.

### SUMMARY OF THE INVENTION

Certain aspects of the present disclosure are described in the appended claims. There are additional features and advantages of the subject matter described herein. They will become apparent as this specification proceeds. The various features described in the claims and below for various embodiments may be used in combination or separately. For example, specified ranges may be inclusive of their recited endpoints, unless explicitly excluded.

According to an embodiment of the invention, a compound having a general Formula (I) is provided: 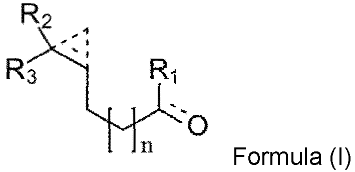
wherein n = 1 or 2;
wherein R¹ = H, methyl, ethyl, or alkoxy; and
wherein R² = H, methyl, or ethyl; and R³ = ethyl, 2,4-dimethylcyclohex-3-enyl, 1-ethylpropyl, 1,5-dimethylhex-4-enyl, cyclopentyl, 1,5-dimethylhexyl, 2,6-dimethylhepta-1,5-dienyl, cyclohexyl, 3,3-dimethylcyclohexyl, 2,6-dimethylhept-5-enyl, 2-phenylpropyl, 4-methylpent-3-enyl, hex-3-enyl, 1,5-dimethyl-5-methoxyhexyl, or 1-methylpropyl; or
wherein R² and R³ taken together form: 4-propylcyclohexyl, 3,3-dimethylcyclohexyl, or 4-methylcyclohexyl.

In accordance with another embodiment of the invention, a fragrant composition is provided comprising at least one compound of general Formula (I), and a solvent is provided.

In accordance with another embodiment of the invention, use of at least one compound of general Formula (I) is provided, said use is to confer, modify or enhance the olfactory properties of a substance, composition, or article.

### DETAILED DESCRIPTION

Unless otherwise explained, all technical and scientific terms used herein have the same meaning as commonly understood by one of ordinary skill in the art to which this disclosure belongs. In case of conflict, the present specification, including explanations of terms, will control. The singular terms "a," "an," "at least one," and "the" include plural referents unless context clearly indicates otherwise. Similarly, the word "or" is intended to include "and" unless the context clearly indicates otherwise. The term "comprising" means "including;" hence, "comprising A or B" means including A or B, as well as A and B together.

Embodiments of the present invention relate to compounds useful as perfume or fragrant ingredients, as well as their preparation process and uses in the chemical industry, and in particular in perfumery, cosmetics, parapharmacy, home and personal care applications, as well as in food, said compounds having interesting organoleptic properties and a particular potency and persistence. As used herein, the term "fragrant" is used here to refer to any organoleptic compound that pleasantly stimulates the sense of smell. The terms "fragrant" or "perfume" are used interchangeably in the present disclosure. Thus, the expressions "fragrant composition" and "perfume composition" may be used interchangeably.

In particular, said compounds exhibit specific odor characteristics and might contribute to muguet (lily of the valley) odor characteristics. Still more particularly, embodiments of the invention relate to perfume compositions that contain no, or substantially no, Lilial^{™}. Embodiments of the invention further relate to methods of making said perfume ingredients and perfume compositions, as well as the use of said perfume ingredients and perfume compositions in fine fragrances and consumer products, such as personal care and household care products. The invention also relates to said fine fragrances and consumer products containing said perfume ingredients or perfume preparations.

In accordance with an embodiment of the invention, a compound having a general Formula (I) is provided: 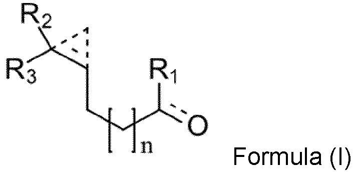
wherein n = 1 or 2;
wherein R¹ = H, methyl, ethyl, or alkoxy; and
wherein R² = H, methyl, or ethyl; and R³ = ethyl, 2,4-dimethylcyclohex-3-enyl, 1-ethylpropyl, 1,5-dimethylhex-4-enyl, cyclopentyl, 1,5-dimethylhexyl, 2,6-dimethylhepta-1,5-dienyl, cyclohexyl, 3,3-dimethylcyclohexyl, 2,6-dimethylhept-5-enyl, 2-phenylpropyl, 4-methylpent-3-enyl, hex-3-enyl, 1,5-dimethyl-5-methoxyhexyl, or 1-methylpropyl; or
wherein R² and R³ taken together form 4-propylcyclohexyl, 3,3-dimethylcyclohexyl, or 4-methylcyclohexyl.

As used herein, alkoxy represents a C1-C5 alkoxy group, such as methoxy, ethoxy, n-propoxy, iso-propoxy, butoxy, sec-butoxy, iso-butoxy, tert-butoxy, pentoxy, sec-pentoxy, iso-pentoxy, neopentoxy, tert-pentoxy, etc.

In an embodiment, the compound having a general Formula (I) is selected from the group consisting of: ethyl 6,10-dimethylundeca-4,9-dienoate; ethyl 6,10-dimethylundec-4-enoate; ethyl 3-(2-(6-methylheptan-2-yl)cyclopropyl)propanoate; 7,11-dimethyldodeca-4,10-dienal; 10-methoxy-6,10-dimethylundec-4-enal; 7,11-dimethyldodec-5-enal; ethyl 5-(4-propylcyclohexylidene)pentanoate; ethyl 6-(3,3-dimethylcyclohexyl)hex-5-enoate; 5-(3,3-dimethylcyclohexyl)hex-4-enal; and 6-ethyloct-4-enal.

According to embodiments of the present invention, use of one or more compounds having a general chemical Formula (I) as a fragrancing agent, a flavoring agent, an odor neutralizing agent, or a taste-masking agent is provided. Moreover, since the compounds are characterized by having a floral, lily-of-the-valley fragrance, the compounds may be used to impart said fragrance characteristics to compositions, meaning that they may be used as a substitute for some or all of any 3-(4-tert-butylphenyl)-2-methylpropanal content. In other words, in an embodiment, the compositions may contain no Lilial^{™} (3-(4-tert-butylphenyl)-2-methylpropanal). In another embodiment, the compositions comprising one or more compounds having a general chemical Formula (I) contains less than 1 ppm.

According to embodiments of the present invention, a composition comprising one or more compound of general Formula (I) is provided. Said composition can be for instance a fragrance composition, a flavoring composition, a pharmaceutical composition, a cosmetic composition, a cleaning composition, a food composition, or an intermediate composition for the preparation of one of said compositions.

In accordance with another embodiment of the invention, a fragrant composition is provided, which comprises a solvent, and at least one compound of general Formula (I), preferably selected from the group consisting of: ethyl 6,10-dimethylundeca-4,9-dienoate; ethyl 6,10-dimethylundec-4-enoate; ethyl 3-(2-(6-methylheptan-2-yl)cyclopropyl)propanoate; 7,11-dimethyldodeca-4,10-dienal; 10-methoxy-6,10-dimethylundec-4-enal; 7,11-dimethyldodec-5-enal; ethyl 5-(4-propylcyclohexylidene)pentanoate; ethyl 6-(3,3-dimethylcyclohexyl)hex-5-enoate; 5-(3,3-dimethylcyclohexyl)hex-4-enal; and 6-ethyloct-4-enal.

Non-limiting examples of solvents useful in fragrant compositions include ethanol, propylene glycol (PG), 1,3-propanediol, dipropylene glycol (DPG), and/or isopropyl myristate.

The effective amount of the compounds of the invention to be incorporated into these compositions depends on the nature of said compositions, the desired odorous or flavoring effect and the nature of other odorous or flavoring compounds possibly present. It is easily determined by those skilled in the art, and can vary in a very wide range, from 100 ppb to 50%. For example, in an embodiment, the at least one compound of general Formula (I) may be incorporated into composition in an amount from 100 ppb to 50%, or from 0.001 to 50%, in particular 0.01 to 30%. The preceding percentages are expressed in total weight of the composition.

Other odorous substances that can be used in combination with the compounds of the present invention may be natural products such as extracts, essential oils, absolutes, resinoids, resins, concretes etc., but also synthetic products such as hydrocarbons, alcohols, aldehydes, ketones, ethers, acids, esters, acetals, nitriles etc., including saturated or unsaturated, aliphatic, heterocyclic, or carbocyclic compounds. Such odorous substances are mentioned, for example, in S. Arctander, "Perfume and Flavor Chemicals" (Montclair, N.J., 1969), or in "Common Fragrance and Flavor Materials", Wiley-VCH, Weinheim, 2006. Finally, a plurality of compounds of general Formula (I) can also be used in combination in the same composition.

In accordance with another embodiment of the invention, the composition is a perfume composition, comprising at least one compound of general Formula (I), preferably selected from the group consisting of: ethyl 6,10-dimethylundeca-4,9-dienoate; ethyl 6,10-dimethylundec-4-enoate; ethyl 3-(2-(6-methylheptan-2-yl)cyclopropyl)propanoate; 7,11-dimethyldodeca-4,10-dienal; 10-methoxy-6,10-dimethylundec-4-enal; 7,11-dimethyldodec-5-enal; ethyl 5-(4-propylcyclohexylidene)pentanoate; ethyl 6-(3,3-dimethylcyclohexyl)hex-5-enoate; 5-(3,3-dimethylcyclohexyl)hex-4-enal; and 6-ethyloct-4-enal.

Because of the pleasant lily-of-the-valley (floral) smell they emit, the compounds of general Formula (I) find many applications in perfumery. The term "perfumery" is used here in its general sense; it refers not only to traditional perfumery (alcoholic or not), but also to other areas in which the smell/fragrance of products is important. Reference may thus be made to perfumery compositions in the usual and traditional sense (such as perfume bases and concentrates, perfumes, colognes, eau de toilette, indoor air fresheners, home fragrances, scented candles and similar products), to topical compositions including cosmetic compositions (such as face and / or body creams, talcum powders, hair oils, shampoos, hair lotions, bath salts and oils, shower and/or bath gels, toilet soaps, antiperspirants and body deodorants, shaving lotions and creams, soaps, toothpastes, mouthwashes, ointments, and similar products), as well as cleaning products, in particular household cleaning products (such as detergents, fabric softeners, home air fresheners, home fragrances and similar products).

It may, in particular, be a composition of the traditional perfumery, a cosmetic composition, cleaning product, or a so-called "intermediate composition", intended to be used for the preparation of compositions or finished products (including perfumes, cosmetics, cleaning products).

Such a perfume composition is generally prepared from a basic product, in which the compound or compounds of the invention are found to be incorporated. The basic product will be easily determined by those skilled in the art according to the composition envisaged and therefore the intended use. The composition of these basic products and the nature of their usual components, such as perfuming ingredient(s), flavoring ingredient(s), solvent(s), additive(s), fixative(s), and / or adjuvant (s), are well known to those skilled in the art.

A fixative is a base ingredient or blend that is utilized in perfumes to help the scent last longer. For example, fixatives can reduce the rate of evaporation of the more volatile materials in fragrance composition. A non-limiting list of fixatives includes resinoids such as benzoin, labdanum, myrrh, olibanum, storax, and tolu balsam; terpenoids such as ambroxide ((3aR,5aS,9aS,9bR)-3a,6,6,9a-tetramethyldodecahydronaphtho[2,1-b]furan); polycyclic ketones such as civetone ((9Z)-cycloheptadec-9-en-1-one) and muscone ((3R)-3-methylcyclopentadecan-1-one); glycerin; Galaxolide (4,6,6,7,8,8-hexamethyl-1,3,4,6,7,8-hexahydrocyclopenta[g]isochromene); Iso E Super (1-(1,2,3,4,5,6,7,8-octahydro-2,3,8,8,-tetramethyl-2-naphthyl)ethan-1-one); Orrisroot (Rhizoma iridis); benzyl benzoate; triethyl citrate; dipropylene glycol (DPG); ethyl hexyl glycerin; and Oud base.

The compounds used in these perfume compositions, in particular the compounds of the invention, may be incorporated into or on an inert support material. The carrier materials that can be used are many and varied, for example polar solvents, oils, greases, finely divided solids, cyclodextrins, maltodextrins, gums, resins, and any other carrier material known for such compositions (for example, soaps, candles, ointments, textiles, wipes, scented gels, or the like).

According to another embodiment of the invention, the composition is a flavor composition comprising: at least one compound of Formula (I), preferably selected from the group consisting of: ethyl 6,10-dimethylundeca-4,9-dienoate; ethyl 6,10-dimethylundec-4-enoate; ethyl 3-(2-(6-methylheptan-2-yl)cyclopropyl)propanoate; 7,11-dimethyldodeca-4,10-dienal; 10-methoxy-6,10-dimethylundec-4-enal; 7,11-dimethyldodec-5-enal; ethyl 5-(4-propylcyclohexylidene)pentanoate; ethyl 6-(3,3-dimethylcyclohexyl)hex-5-enoate; 5-(3,3-dimethylcyclohexyl)hex-4-enal; and 6-ethyloct-4-enal.

For example, in one aspect, the flavor composition may be an ingestible product, which refers to a "beverage", "food", an "edible composition", and/or a "food product". Said ingestible product preferentially relates, but is not limited to, products intended for human consumption, intended for animal feed (pets) or pharmaceutical compositions. Examples of food products may include, but are not limited to, snacks, confectionery, plant materials, and meals that may or may not provide essential nutrients. Plant materials include cocoa, cocoa beans, coffee, coffee beans, and tea leaves or powder. Examples of food products include salad dressings, sauces, sauces, marinades, sticks, nutrition bars, pastries, breads, caramel, cooked cereals, meat products, poultry products, meat, poultry, poultry, fish, marine protein sources, beans, pasta, confectionery products, salty snacks, dairy products, cheeses, yogurt, butter, margarine, ready-to-eat cereals, condiments and sauces and beverages. In particular, the term "beverage" includes mixtures and concentrates, including, but not limited to, ready-to-drink alcoholic and non-alcoholic beverages and dry powdered beverages. Non-limiting examples of beverages include, but are not limited to, soft drinks, brewed beverages, dairy products, drinking yogurt, milk, coffee whitening agents, nutritional drinks, and the like. Non-limiting examples of animal feed may include, but are not limited to, pet food, such as dogs and cats; rodent food; livestock feed; cattle feed; horse feed; and the like. Other applications for the compounds of general Formula (I) may also include tobacco products or tobacco-related products.

In accordance with yet another embodiment of the invention, the use of at least one compound of general Formula (I), in a form of a stereoisomer or a mixture of stereoisomers, or a racemic mixture, to confer, modify, or enhance the organoleptic properties of a substance, a composition, or an article is provided.

The present invention also relates to a process for modifying the organoleptic properties of a substance, a composition or an article comprising at least one of the following steps: adding a compound of general Formula (I) to said substance, composition, or article, or applying a compound of Formula (I) to the surface of said article. As used herein, "organoleptic properties" means any property likely to modify, improve or enhance the organoleptic perception of a substance, composition or article by a user (e.g. olfactory or gustatory perception)

In an embodiment, at least one compound of Formula (I) is used as a fragrant agent, alone or in combination with at least one other odorant substance, and/ or at least one solvent, and / or at least one adjuvant. The additional odorant agent(s), solvents and adjuvants are known to those skilled in the art who will be able to choose the most appropriate or the most appropriate according to the desired effect.

The compounds according to the invention may particularly be used as a masking agent or as an odor neutralizing agent. As used herein, the term "masking agent" or "odor neutralizing agent" is meant to identify a characteristic of the compounds within the scope of the invention to reduce or eliminate the perception of a bad odor generated by one or more other molecules present in a composition or a product.

In particular, the compounds according to the invention can be used alone or in combination with taste-modulating compounds. "Taste-modulating compound" refers to a compound which can modify taste and sensory perceptions. In any case, the specificity of such taste-modulating compounds is that they do not exhibit noticeable taste and flavor properties (without taste and flavor). Such flavor-altering compounds can be of synthetic or natural origin.

### EXAMPLES

The following examples illustrate a particular way of preparing the compounds of the invention, as well as the olfactory / aromatic profile of each of the exemplified compounds. These examples are for illustrative purposes only and should not be understood as limiting the general scope of the invention. Unless stated otherwise, percentages (%) are based on weight.

**Compound 1** (Ethyl 6,10-dimethylundeca-4,9-dienoate): 134 g of [3-(ethoxycarbonyl)propyl]triphenylphosphonium bromide and 420 ml of tetrahydrofuran (THF) were charged to a 3-necked flask fitted with a thermometer, a stopper, and a condenser. The reaction mixture was stirred with a mechanical stirrer at room temperature. 110 ml of 20%wt. solution of sodium ethoxide in ethanol are added drop wise. After addition, the reaction mixture is stirred for 1 hour at 40 °C. Then, 29.4 g of 2,6-dimethylhept-5-enal were added. After 1 night at 40 °C, the reaction was considered complete. A solution of sulfuric acid was added to the reaction mixture (200 ml of water + 8 ml of H₂SO₄). After decantation, the aqueous layer was extracted with methyl tert-butyl ether (MTBE) (200 mL). The mixture was decanted and the combined organic phases were washed with brine, dried over MgSO₄, filtered and concentrated under vacuum. The crude product was distilled under reduced pressure (boiling point: 82 °C at 0.1 Torr) to provide clean ethyl 6,10-dimethylundeca-4,9-dienoate.
Olfactory description: fruity, musky, clean. Compound 1 has the following spectral characteristics:
¹H-NMR (300 MHz, CDCl₃): δ (ppm) 5.39 - 5.02 (m, 3H), 4.13 (q, *J =* 7.1 Hz, 2H), 2.55 - 2.25 (m, 5H), 2.02 - 1.79 (m, 2H), 1.67 (q, *J* = 1.3 Hz, 3H), 1.58 (d, *J* = 1.3 Hz, 3H), 1.42 - 1.23 (m, 1H), 1.28 (s, 1H), 1.28 - 1.11 (m, 3H), 0.93 (d, *J* = 6.7 Hz, 3H).
¹³C-NMR (75 MHz, CDCl₃): δ (ppm) 173.22, 137.62, 137.56, 131.25, 131.12, 128.98, 126.36, 126.13, 124.71, 124.65, 122.65, 77.24, 60.26, 60.19, 43.73, 37.52, 37.08, 36.25, 34.58, 34.50, 31.40, 27.95, 25.98, 25.76, 25.67, 23.06, 22.50, 21.26, 20.73, 17.63, 14.22.

**Compound 2** (Ethyl 6,10-dimethylundec-4-enoate): 114 g of [3-(ethoxycarbonyl)propyl]triphenylphosphonium bromide and 356 ml of THF were charged to a 3-necked flask fitted with a thermometer, a stopper, and a condenser. The reaction mixture was stirred with a mechanical stirrer at room temperature. 93 ml of 20%wt. solution of sodium ethoxide in ethanol are added drop wise. After addition, the reaction mixture is stirred for 1 hour at 50 °C. Then, 25.3 g of 2,6-dimethylheptanal were added. After 1 hour, the reaction was considered complete. A solution of sulfuric acid was added to the reaction mixture (200 ml of water + 8 ml of H₂SO₄). After decantation, the aqueous layer was extracted with MTBE (200 mL). The mixture was decanted and the combined organic phases were washed with brine, dried over MgSO4, filtered and concentrated under vacuum. The crude product was distilled under reduced pressure (boiling point: 80 °C at 0.2 Torr) to provide clean ethyl 6,10-dimethylundec-4-enoate.
Olfactory description: fruity, red fruits, floral, musky. Compound 2 has the following spectral characteristics:
¹H-NMR (300 MHz, CDCl₃): δ (ppm) 4.12 (q, *J* = 7.1 Hz, 2H), 2.45 - 2.30 (m, 2H), 1.95 (dddd, *J =* 14.8, 12.2, 7.7, 4.6 Hz, 1H), 1.52 (dtd, *J =* 13.3, 6.6, 2.0 Hz, 1H), 1.43 - 1.06 (m, 6H), 1.05 - 0.91 (m, 4H), 0.86 (d, *J* = 6.6 Hz, 7H), 0.82 - 0.41 (m, 3H), 0.51 (s, 1H), -0.17 - -0.35 (m, 1H).
¹³C-NMR (75 MHz, CDCl₃): δ (ppm) 173.88, 60.16, 39.37, 39.33, 38.15, 37.88, 34.99, 34.75, 33.31, 32.90, 29.71, 27.97, 27.95, 24.99, 24.87, 24.58, 24.26, 24.19, 24.03, 23.70, 22.73, 22.71, 22.60, 22.58, 20.60, 20.19, 16.30, 14.99, 14.24, 10.42, 9.60.

**Compound 3** (Ethyl 3-(2-(6-methylheptan-2-yl)cyclopropyl) propanoate): 18 g of zinc, 1.8 g of copper(I) chloride and 100 ml of cyclopropyl methyl ether (CPME) were charged to a 3-necked flask fitted with a thermometer, a stopper, and a condenser. The reaction mixture was stirred at room temperature. 0.65 ml of acetyl chloride and 7.38 ml of diiodomethane were added. The reaction mixture was stirred 30 minutes at 60 °C. Then, 22 g of Compound 2 were added at room temperature dropwise. Finally, 14.8 ml of diiodomethane in 30 ml of cyclopentyl methyl ether (CPME) were added and the reaction mixture was stirred at room temperature for 3 days. A saturated aqueous solution of ammonium chloride was added to the reaction mixture at -10 °C (100 ml). After filtration and decantation, the organic phase was washed with brine, dried over MgSO₄, filtered and concentrated under vacuum. The crude product was distilled under reduced pressure (boiling point: 92 °C at 0.1 Torr) to provide clean ethyl 3-(2-(6-methylheptan-2-yl)cyclopropyl)propanoate.
Olfactory description: fruity, musky, powdery. Compound 3 has the following spectral characteristics:
¹H-NMR (300 MHz, CDCl₃): δ (ppm) 4.12 (q, *J* = 7.1 Hz, 2H), 2.45 - 2.30 (m, 2H), 1.95 (dddd, *J =* 14.8, 12.2, 7.7, 4.6 Hz, 1H), 1.52 (dtd, *J =* 13.3, 6.6, 2.0 Hz, 1H), 1.43 - 1.06 (m, 7H), 1.05 - 0.92 (m, 4H), 0.86 (d, *J* = 6.6 Hz, 8H), 0.82 - 0.65 (m, 1H), 0.71 - 0.41 (m, 3H), 0.51 (s, 1H).
¹³C-NMR (75 MHz, CDCl₃): δ (ppm) 173.83, 173.58, 60.13, 39.36, 39.33, 38.14, 37.88, 37.52, 37.40, 34.97, 34.74, 33.30, 32.89, 29.70, 27.96, 27.94, 24.97, 24.85, 24.57, 24.25, 24.19, 24.02, 23.69, 22.71, 22.68, 22.58, 22.57, 20.58, 20.18, 16.29, 15.37, 14.99, 14.22, 10.41, 9.59.

**Compound 4** (7,11-Dimethyldodeca-4,10-dienal): 132 g of (4-carboxybutyl)triphenylphosphonium bromide and 594 ml of toluene were charged to a 3-necked flask fitted with a thermometer, a stopper, and a condenser. The reaction mixture was stirred with a mechanical stirrer at room temperature. 73.4 g of potassium tert-butoxide are added portion wise. After addition, the reaction mixture is stirred for 2 hours at RT. Then, 50 g of 2,6-dimethylhept-5-enal were added at a rate such that the temperature was easily controlled using a water bath (20-30 °C). After 4 hours at RT, the reaction was considered complete. Water was added to the reaction mixture (500 ml). After decantation, the organic layer was discarded. The aqueous layer was acidified using H₂SO₄ until pH is at 2-3 and extracted with MTBE (500 mL). The mixture was decanted and the aqueous layer was discarded. The organic layer was washed with brine, dried over MgSO₄, filtered and concentrated under vacuum. The crude product containing carboxylic acid intermediate was used in the next step without any further purification.

In a 3-necked flask fitted with a thermometer, a condenser and magnetic stirring were placed under nitrogen atmosphere 52 g of the carboxylic acid intermediate in 218 ml of dichloromethane. Then, 53 g of N,N'-carbonyldiimidazole were portion wise. After 2 h stirring at RT, 31.9 g of N,O-dimethylhydroxylamine hydrochloride were added and the mixture was stirred for 3 days at RT. Then, 100 ml of aqueous HCl 10% were added. After decantation, the organic layer was washed with aq. bicarbonate, then Brine, dried over MgSO₄ then concentrated under vacuum. The crude product containing Weinreb amide intermediate was used in the next step without any further purification.

In a 3-necked flask under nitrogen atmosphere fitted with a thermometer, an addition funnel and a condenser under magnetic stirring were placed 10 g of the Weinreb amide intermediate in 37.4 ml of toluene. At 0 °C, 37.4 ml of Diisobutylaluminum hydride solution 1.0 M in THF were added dropwise. After 2 hours at RT, the reaction mixture is poured into a cold HCl 10% aqueous solution (100 ml). After decantation, the organic phase was washed with saturated aqueous sodium bicarbonate solution (100 ml), then Brine (100 ml), dried over MgSO₄ then concentrated under vacuum. A vacuum distillation was performed on the crude product under 105 °C under 0.6 Torr to afford clean 7,11-dimethyldodeca-4,10-dienal.
Olfactory description: aldehydic, green, floral, aqueous, powerful. Compound 4 has the following spectral characteristics:
¹H-NMR (300 MHz, CDCl₃): δ (ppm) 5.45 - 5.24 (m, 2H), 5.02 (ddt, *J* = 8.5, 7.1, 1.4 Hz, 1H), 2.55 - 2.30 (m, 1H), 2.34 - 2.22 (m, 1H), 2.27 (s, 1H), 2.14 - 1.75 (m, 3H), 1.71 - 1.50 (m, 6H), 1.50 - 1.18 (m, 2H), 1.23 - 0.96 (m, 3H), 0.96 -0.74 (m, 3H), 0.79 (s, 1H).
¹³C-NMR (75 MHz, CDCl₃): δ (ppm) 202.31, 130.26, 127.84, 124.77, 77.45, 77.23, 77.03, 76.61, 43.80, 36.74, 34.44, 32.93, 25.71, 25.66, 20.19, 19.47, 17.64.

**Compound 5** (10-Methoxy-6,10-dimethylundec-4-enal): 99 g of [3-(ethoxycarbonyl)propyl]triphenylphosphonium bromide and 330 ml of THF were charged to a 3-necked flask fitted with a thermometer, a stopper, and a condenser. The reaction mixture was stirred with a mechanical stirrer at room temperature. 87 ml of 20%wt. solution of sodium ethoxide in ethanol are added drop wise. After addition, the reaction mixture is stirred for 2 hours at RT. Then, 28.7 g of 6-methoxy-2,6-dimethylheptanal were added. After 1 night at 50 °C, the reaction was considered complete. A solution of sulfuric acid was added to the reaction mixture (200 ml of water + 8 ml of H₂SO₄). After decantation, the aqueous layer was extracted with MTBE (200 mL). The mixture was decanted and the combined organic phases were washed with brine, dried over MgSO₄, filtered and concentrated under vacuum. The crude product was distilled under reduced pressure (boiling point: 102 °C at 0.1 Torr) to provide clean ester intermediate.

In a first 3-necked flask under nitrogen atmosphere fitted with a thermometer, an addition funnel and a condenser under magnetic stirring were placed 35.9 ml of sodium bis(2-methoxy)aluminum hydride 70% wt. in toluene in 30.9 ml of MTBE. At RT, 15.85 ml of 2,6-dimethylmorpholine were added dropwise and this mixture was stirred at RT for 2 h. In a second 3-necked flask under nitrogen atmosphere fitted with a thermometer, an addition funnel and a condenser under magnetic stirring were placed 11.6 g of the ester intermediate in 88 ml of MTBE. At -20 °C, the solution prepared in the first flask was added dropwise. At the end of the addition, the reaction mixture is poured into a cold HCl 10% aqueous solution (100 ml). After decantation, the organic phase was washed with saturated aqueous sodium bicarbonate solution (100 ml), then Brine (100 ml), dried over MgSO₄ then concentrated under vacuum. A vacuum distillation was performed on the crude product under 80 °C under 0.8 Torr to afford clean 10-methoxy-6,10-dimethylundec-4-enal.
Olfactory description: aldehydic, floral, lily of the valley, marine, powerful. Compound 5 has the following spectral characteristics:
¹H-NMR (300 MHz, CDCl₃): δ (ppm) 9.74 (t, *J =* 1.6 Hz, 1H), 5.37 (dtd, *J* = 10.8, 7.1, 0.9 Hz, 2H), 5.06 (tt, *J* = 10.9, 1.5 Hz, 2H), 2.53 - 2.26 (m, 5H), 2.19 - 2.00 (m, 2H), 1.40 (dqd, *J* = 13.1, 7.4, 4.7 Hz, 3H), 1.27 - 1.01 (m, 3H), 0.80 (t, *J =* 7.4 Hz, 8H).
¹³C-NMR (75 MHz, CDCl₃): δ (ppm) 202.39, 202.15, 136.15, 136.06, 135.16, 134.97, 129.16, 129.09, 127.80, 127.27, 77.27, 62.54, 46.22, 43.99, 43.62, 40.74, 40.62, 32.83, 30.05, 28.23, 28.13, 27.89, 27.61, 25.26, 24.06, 20.49, 11.81, 11.66.

**Compound 6** (7,11-dimethyldodec-5-enal): 607.7 g of (4-carboxybutyl)triphenylphosphonium bromide and 1092 ml of toluene were charged to a 3-necked flask fitted with a thermometer, a stopper, and a condenser. The reaction mixture was stirred with a mechanical stirrer at room temperature. 338.4 g of potassium tert-butoxide are added portion wise. After addition, the reaction mixture is stirred for 2 hours at RT. Then, 195 g of 2,6-dimethylheptanal were added at a rate such that the temperature was easily controlled using a water bath (20-30 °C). After 1 night at RT, the reaction was considered complete. Water was added to the reaction mixture (500 ml). After decantation, the organic layer was discarded. The aqueous layer was acidified using H₂SO₄ until pH is at 2-3 and extracted with MTBE (500 mL). The mixture was decanted and the aqueous layer was discarded. The organic layer was washed with brine, dried over MgSO₄, filtered and concentrated under vacuum. The crude product was distilled under reduced pressure (boiling point: 143 °C at 0.6 Torr) to provide 143 g of carboxylic acid intermediate.

In a 2 L flask fitted with a thermometer, a condenser and magnetic stirring were placed under nitrogen atmosphere 24 g of LiAlH₄ and 631 ml of THF. Then, 170 g of the carboxylic acid intermediate was added through the dropping funnel over a period of 1h30. After 1 hour, HCl 34% was added carefully added until the aluminum salts are effectively sticked to the flask. The suspension is filtered and dried under vacuum without any additional washings to afford 129 g of clear oil containing alcohol intermediate.

In a 2 L flask fitted with a thermometer and a condenser under magnetic stirring were successively added 129 g of the alcohol intermediate, 4.7 g of (2,2,6,6-tetramethylpiperidin-1-yl)oxy, 202 ml of water and 405 ml of dichloromethane. At 0 °C, 215.2 g of (diacetoxyiodo)benzene portion wise. After 1 h, the reaction was complete and showed iodobenzene as a by-product and about 10% of overoxidation product (carboxylic acid). First the reaction mixture was decanted and the aqueous layer is discarded. The organic phase was washed with water (100 mL), then HCl 10%, then aq. thiosulfate under mixing for 15 minutes until a KI / Starch peroxide test is negative. After decantation, the organic layer was washed with aq. bicarbonate, then Brine, dried over MgSO₄ then concentrated under vacuum to afford 243 g or crude mixture. Flash vacuum distillation is performed under 135 °C under 0.6 Torr to afford 40 g of clean 7,11-dimethyldodec-5-enal. Olfactory description: marine, aldehydic, green, very powerful. Compound 6 has the following spectral characteristics:
¹H-NMR (300 MHz, CDCl₃): δ (ppm) 9.79 (s, 1H), 5.35 - 5.06 (m, 2H), 2.55 - 2.27 (m, 3H), 2.17 - 1.92 (m, 2H), 1.78 - 1.64 (m, 2H), 1.62 - 1.44 (m, 2H), 1.34 - 1.03 (m, 6H), 0.99 - 0.80 (m, 9H).
¹³C-NMR (75 MHz, CDCl₃): δ (ppm) 202.56, 137.79, 126.68, 43.33, 39.11, 37.69, 31.73, 27.93, 26.73, 25.24, 22.66, 22.61, 22.20, 21.33.

**Compound 7** (Ethyl 5-(4-propylcyclohexylidene)pentanoate): 242 g of (4-carboxybutyl)triphenylphosphonium bromide and 1092 ml of toluene were charged to a 3-necked flask fitted with a thermometer, a stopper, and a condenser. The reaction mixture was stirred with a mechanical stirrer at room temperature. 135 g of potassium tert-butoxide are added portion wise. After addition, the reaction mixture is stirred for 2 hours at RT. Then, 77 g of 4-propylcyclohexan-1-one were added at a rate such that the temperature was easily controlled using a water bath (20-30 °C). After 1 night at RT, the reaction was considered complete. Water was added to the reaction mixture (500 ml). After decantation, the organic layer was discarded. The aqueous layer was acidified using H₂SO₄ until pH is at 2-3 and extracted with MTBE (500 mL). The mixture was decanted and the aqueous layer was discarded. The organic layer was washed with brine, dried over MgSO₄, filtered and concentrated under vacuum. The crude product was distilled under reduced pressure (boiling point: 147 °C at 0.6 Torr) to provide 91 g of carboxylic acid intermediate.

In a 100 mL flask fitted with a thermometer, a condenser and magnetic stirring were placed 20.5 g of ethanol, 0.12 ml of sulfuric acid and 10 g of the carboxylic acid intermediate. This mixture is heated at 60 °C overnight. Water was added to the reaction mixture (50 ml). After decantation, the organic layer was washed with an aqueous sodium bicarbonate saturated solution, and with Brine, then dried over MgSO₄, filtered and concentrated under vacuum. The crude product was distilled under reduced pressure (boiling point: 124 °C at 0.6 Torr) to provide 10 g of ethyl 5-(4-propylcyclohexylidene)pentanoate.
Olfactory description: floral, aqueous, green. Compound 7 has the following spectral characteristics:
¹H-NMR (300 MHz, CDCl₃): δ (ppm) 5.41 - 5.31 (m, 1H), 4.12 (q, *J =* 7.1 Hz, 2H), 2.29 (td, *J* = 7.4, 3.8 Hz, 2H), 2.15 - 1.98 (m, 1H), 2.02 - 1.84 (m, 4H), 1.84 - 1.51 (m, 4H), 1.51 - 1.08 (m, 10H), 0.89 (td, *J* = 7.2, 2.8 Hz, 4H).
¹³C-NMR (75 MHz, CDCl₃): δ (ppm) 173.81, 140.70, 137.23, 120.64, 120.03, 77.23, 60.14, 39.06, 38.85, 37.54, 37.26, 36.45, 34.84, 34.27, 34.04, 33.69, 33.24, 32.04, 29.37, 28.30, 27.90, 27.16, 26.49, 25.32, 24.65, 20.11, 20.06, 14.35, 14.24.

**Compound 8** (ethyl 6-(3,3-dimethylcyclohexyl)hex-5-enoate): 107 g of (4-carboxybutyl)triphenylphosphonium bromide and 485 ml of toluene were charged to a 3-necked flask fitted with a thermometer, a stopper, and a condenser. The reaction mixture was stirred with a mechanical stirrer at room temperature. 59.9 g of potassium tert-butoxide are added portion wise. After addition, the reaction mixture is stirred for 2 hours at RT. Then, 34 g of 3,3-dimethylcyclohexane-1-carbaldehyde were added at a rate such that the temperature was easily controlled using a water bath (20-30 °C). After 1 night at RT, the reaction was considered complete. Water was added to the reaction mixture (500 ml). After decantation, the organic layer was discarded. The aqueous layer was acidified using H₂SO₄ until pH is at 2-3 and extracted with MTBE (500 mL). The mixture was decanted and the aqueous layer was discarded. The organic layer was washed with brine, dried over MgSO₄, filtered and concentrated under vacuum. The crude product was (57 g) containing the carboxylic acid intermediate was used without any further purification in the next step.

In a 500 mL flask fitted with a thermometer, a condenser and magnetic stirring were placed 117 g of ethanol, 1.2 g of sulfuric acid and 57 g of the carboxylic acid intermediate. This mixture is heated at 60 °C overnight. Water was added to the reaction mixture (50 ml). After decantation, the organic layer was washed with an aqueous sodium bicarbonate saturated solution, and with Brine, then dried over MgSO₄, filtered and concentrated under vacuum. The crude product was distilled under reduced pressure (boiling point: 109 °C at 0.5 Torr) to provide 46 g of pure ethyl 6-(3,3-dimethylcyclohexyl)hex-5-enoate.
Olfactory description: floral, powdery, musky, red fruits. Compound 8 has the following spectral characteristics:
¹H-NMR (300 MHz, CDCl₃): δ (ppm) 5.34 - 5.10 (m, 2H), 4.13 (q, *J* = 7.1 Hz, 2H), 2.40 (tdt, *J=* 12.1, 7.7, 3.8 Hz, 1H), 2.33 - 2.27 (m, 2H), 2.15 - 2.02 (m, 2H), 1.76 - 1.63 (m, 2H), 1.63 - 1.30 (m, 4H), 1.26 (t, *J* = 7.1 Hz, 4H), 1.14 - 1.01 (m, 1H), 0.98 - 0.80 (m, 8H).
¹³C-NMR (75 MHz, CDCl₃): δ (ppm) 173.68, 137.24, 126.60, 60.18, 46.29, 38.73, 33.73, 33.41, 33.08, 32.06, 30.56, 26.75, 25.08, 24.64, 22.02, 14.24.

**Compound 9** (5-(3,3-Dimethylcyclohexyl)hex-4-enal): 500 g of (3-carboxypropyl)triphenylphosphonium bromide and 1165 ml of toluene were charged to a 3-necked flask fitted with a thermometer, a stopper, and a condenser. The reaction mixture was stirred with a mechanical stirrer at room temperature. 287.5 g of potassium tert-butoxide are added portion wise. After addition, the reaction mixture is stirred for 2 hours at RT. Then, 201 ml of 1-(3,3-dimethylcyclohexyl)ethan-1-one were added at a rate such that the temperature was easily controlled using a water bath (20-30 °C). After 1 night at RT, the reaction was considered complete. Water was added to the reaction mixture (500 ml). After decantation, the organic layer was discarded. The aqueous layer was acidified using H₂SO₄ until pH is at 2-3 and extracted with MTBE (500 mL). The mixture was decanted and the aqueous layer was discarded. The organic layer was washed with brine, dried over MgSO₄, filtered and concentrated under vacuum. The crude product was distilled under reduced pressure (boiling point: 138 °C at 0.8 Torr) to provide 180 g of carboxylic acid intermediate.

In a 2 L flask fitted with a thermometer, a condenser and magnetic stirring were placed under nitrogen atmosphere 28.7 g of LiAlH₄ and 758 ml of THF. Then, 170 g of the carboxylic acid intermediate was added through the dropping funnel over a period of 1h30. After 1 hour, HCl 34% was added carefully added until the aluminum salts are effectively sticked to the flask. The suspension is filtered and dried under vacuum without any additional washings to afford 136 g of clear oil containing alcohol intermediate.

In a 2 L flask fitted with a thermometer and a condenser under magnetic stirring were successively added 136 g of the alcohol intermediate, 5 g of (2,2,6,6-tetramethylpiperidin-1-yl)oxy, 215 ml of water and 413 ml of dichloromethane. At 0 °C, 229 g of (diacetoxyiodo)benzene portion wise. After 1 h, the reaction was complete and showed iodobenzene as a by-product and about 10% of overoxidation product (carboxylic acid). First the reaction mixture was decanted and the aqueous layer is discarded. The organic phase was washed with water (100 mL), then HCl 10%, then aq. thiosulfate under mixing for 15 minutes until a KI / Starch peroxide test is negative. After decantation, the organic layer was washed with aq. bicarbonate, then Brine, dried over MgSO₄ then concentrated under vacuum to afford 262 g or crude mixture. Flash vacuum distillation is performed under 95 °C under 0.5 Torr to afford 78 g of clean 5-(3,3-dimethylcyclohexyl)hex-4-enal.
Olfactory description: floral, lily of the valley, ozonic, green, powerful. Compound 9 has the following spectral characteristics:
¹H-NMR (300 MHz, CDCl₃): δ (ppm) 9.76 (t, *J=* 1.8 Hz, 1H), 5.15 - 4.96 (m, 1H), 2.62 (tt, *J* = 12.0, 3.7 Hz, 1H), 2.46 (tt, *J* = 7.3, 1.6 Hz, 2H), 2.40 - 2.23 (m, 2H), 1.68 - 1.52 (m, 4H), 1.57 - 1.41 (m, 1H), 1.46 - 1.12 (m, 3H), 1.17 - 1.04 (m, 2H), 1.09- 0.87 (m, 7H).
¹³C-NMR (75 MHz, CDCl₃): δ (ppm) Major Isomer (Z) 202.60, 141.76, 121.71, 77.46, 77.04, 76.62, 44.24, 43.85, 38.87, 34.78, 33.59, 33.56, 30.83, 30.60, 24.65, 24.62, 22.40, 19.98, 19.42. Minor Isomer (E) 202.74, 142.07, 119.98, 44.73, 44.00, 42.47, 39.05, 33.59, 31.59, 30.89, 24.65, 22.49, 20.72, 14.44.

**Compound 10** (6-ethyloct-4-enal): 180 g of [3-(ethoxycarbonyl)propyl]triphenylphosphonium bromide and 605 ml of THF were charged to a 3-necked flask fitted with a thermometer, a stopper, and a condenser. The reaction mixture was stirred with a mechanical stirrer at room temperature. 158 ml of 20%wt. solution of sodium ethoxide in ethanol are added drop wise. After addition, the reaction mixture is stirred for 2 hours at RT. Then, 30.3 g of 2-ethylbutanal were added. After 1 hour at RT, the reaction was considered complete. A solution of sulfuric acid was added to the reaction mixture (200 ml of water + 8 ml of H₂SO₄). After decantation, the aqueous layer was extracted with MTBE (200 mL). The mixture was decanted and the combined organic phases were washed with brine, dried over MgSO₄, filtered and concentrated under vacuum. The crude product was distilled under reduced pressure (boiling point: 54 °C at 0.1 Torr) to provide clean ester intermediate.

In a first 3-necked flask under nitrogen atmosphere fitted with a thermometer, an addition funnel and a condenser under magnetic stirring were placed 63.3 ml of sodium bis(2-methoxy)aluminum hydride 70% wt. in toluene in 55 ml of MTBE. At RT, 28 ml of 2,6-dimethylmorpholine were added dropwise and this mixture was stirred at RT for 2 h. In a second 3-necked flask under nitrogen atmosphere fitted with a thermometer, an addition funnel and a condenser under magnetic stirring were placed 15 g of the ester intermediate in 157 ml of MTBE. At -20 °C, the solution prepared in the first flask was added dropwise. At the end of the addition, the reaction mixture is poured into a cold HCl 10% aqueous solution (100 ml). After decantation, the organic phase was washed with saturated aqueous sodium bicarbonate solution (100 ml), then Brine (100 ml), dried over MgSO₄ then concentrated under vacuum. A vacuum distillation was performed on the crude product under 52 °C under 0.8 Torr to afford clean 6-ethyloct-4-enal. Olfactory description: fruity, green, citrus, powerful. Compound 10 has the following spectral characteristics:
¹H-NMR (300 MHz, CDCl₃): δ (ppm) 9.73 (t, *J* = 5.7 Hz, 1H), 5.58 (dt, *J* = 14.4, 5.7 Hz, 1H), 5.37 (ddt, *J* = 14.4, 7.5, 1.4 Hz, 1H), 2.47 - 2.39 (m, 2H), 2.26 - 2.18 (m, 2H), 1.92 (ddddd, *J* = 9.4, 8.0, 6.6, 4.1, 2.5 Hz, 1H), 1.48 (dp, *J* = 11.7, 6.9 Hz, 2H), 1.23 (dp, *J =* 11.5, 6.8 Hz, 2H), 0.85 (td, *J* = 6.9, 1.5 Hz, 6H).
¹³C-NMR (75 MHz, CDCl₃): δ (ppm) 200.91, 135.25, 130.52, 46.89, 42.00, 27.41, 25.18, 11.89.

**Example 1: Pear accord:** 3% DPG of the pear accord 1A was replaced with 3% of select compounds (2, 5, 7, and 8) and the different corresponding accords were applied at 0.6% in a shampoo base and at 1% in concentrated fabric softener base.

**Table 1: Example 1 - Pear accords dosed with Compounds 2, 5, 7, or 8.**

| | Accord 1A | Inventive Accord 1B | Inventive Accord 1C | Inventive Accord 1D | Inventive Accord 1E |
|---|---|---|---|---|---|
| GAMMA UNDECALACTONE | 200 | 200 | 200 | 200 | 200 |
| HEXYL ACETATE | 180 | 180 | 180 | 180 | 180 |
| HABANOLIDE^{™} | 180 | 180 | 180 | 180 | 180 |
| VERDOX^{™} 50%DPG | 172 | 172 | 172 | 172 | 172 |
| SIAMWOOD | 80 | 80 | 80 | 80 | 80 |
| GERANYL ACETATE | 40 | 40 | 40 | 40 | 40 |
| GERANIUM BOURBON EO | 20 | 20 | 20 | 20 | 20 |
| ETHYL METHYLVALERATE | 14 | 14 | 14 | 14 | 14 |
| PRENYL ACETATE | 12 | 12 | 12 | 12 | 12 |
| ISOAMYL ACETATE | 10 | 10 | 10 | 10 | 10 |
| TRIPLAL^{™} | 5 | 5 | 5 | 5 | 5 |
| STYRALLYL ACETATE | 5 | 5 | 5 | 5 | 5 |
| MELONAL^{™} 10% DPG | 1 | 1 | 1 | 1 | 1 |
| CIS 3 HEXENYL CIS 3 HEXENOATE | 1 | 1 | 1 | 1 | 1 |
| DIPROPYLENE GLYCOL - DPG | 80 | 50 | 50 | 50 | 50 |
| Compound 2 | - | 30 | - | - | - |
| Compound 5 | - | - | 30 | - | - |
| Compound 7 | - | - | - | 30 | - |
| Compound 8 | - | - | - | - | 30 |

**Shampoo:** Accord 1A is a pleasant pear accord, a bit juicy, sweet and with musky undertones. At 0.6% in a shampoo base, the accord lacks a bit of strength, and the smell of the base is not fully covered. Inventive accord 1B (compound 2) pushed the musky note, rendering the note more cosmetic, and with a good covering of the smell of the base. Inventive accord 1C (compound 5) renders the accord more apple, very crunchy, juicy fruity, powerful and with sweet-sugary effects. Inventive accord 1D (compound 7) did not provide as big of an impact as with Inventive accord 1B (compound 2). However, Inventive accord 1D is more powerful and a bit muskier, and it covers the smell of the base. Inventive accord 1E (compound 8) gives strength to the accord, which is more fruity, with a juicy flesh, sweet-sugary effect.

**Softener:** Accord 1A at 1% in the base has a similar evaluation as in shampoo base, but with a better coverage of the smell of the base. Inventive accord 1B (compound 2) pushes the musky note, and the accord is more powerful and more pear-apple. Inventive accord 1C (compound 5) provided a similar evaluation as in the shampoo base. Inventive accord 1D (compound 7) is also more musky, and a bit less pear, and it has a good impact. Inventive accord 1E (compound 8), in addition to the remarkable effects shown in shampoo base, gives another dimension to the fruity note, rendering it more complex, a bit more exotic.

**Example 2: Rose accord:** 1.5% DPG of the Rose accord 2A was replaced with 1.5% of select compounds (2, 5, 7, and 8) and the different corresponding accords were applied at 0.6% in a shampoo base and at 1% in concentrated fabric softener base.

**Table 2: Example 2 - Rose accords dosed with Compounds 2, 5, 7, or 8.**

| | Accord 2A | Inventive Accord 2B | Inventive Accord 2C | Inventive Accord 2D | Inventive Accord 2E |
|---|---|---|---|---|---|
| PHENYLETHYL ALCOHOL | 400 | 400 | 400 | 400 | 400 |
| CITRONELLOL | 290 | 290 | 290 | 290 | 290 |
| GERANIOL | 200 | 200 | 200 | 200 | 200 |
| NEROL | 50 | 50 | 50 | 50 | 50 |
| GERANYL ACETATE | 20 | 20 | 20 | 20 | 20 |
| EUGENOL | 10 | 10 | 10 | 10 | 10 |
| CITRAL | 10 | 10 | 10 | 10 | 10 |
| ROSE OXIDE | 5 | 5 | 5 | 5 | 5 |
| DIPROPYLENE GLYCOL - DPG | 15 | - | - | - | - |
| Compound 2 | - | 15 | - | - | - |
| Compound 5 | - | - | 15 | - | - |
| Compound 7 | - | - | - | 15 | - |
| Compound 8 | - | - | - | - | 15 |

**Shampoo:** Accord 2A is a classical rose accord, like a fresh flower, a bit petaly and fruity, somehow green and with a present rose oxide undertone. Inventive accord 2B (compound 2) renders the note more pleasant, with more floralcy, pushing the petaly effect. Inventive accord 2C (compound 5) renders the accord more fruity, giving a nice rosy and more comfortable note. Inventive accord 2D (compound 7) gives more strength to the note, with some more citronella undertone. Inventive accord 2E (compound 8) gives strength to the accord, which is more fruity, with a juicy flesh, sweet-sugary effect.

**Softener** : Accord A at 1% in the softener base has a similar evaluation as in shampoo base, but with a better coverage of the smell of the base. Inventive accord 2B (compound 2) gave a similar effect as it did in the shampoo base. Inventive accord 2C (compound 5) adds fruitiness to the note, but less perceivable than in the shampoo base. Inventive accord 2D (compound 7) has a slight effect on the rose oxide note, which is less harsh. Inventive accord 2E (compound 8) gave a similar effect as it did in the shampoo base.

**Example 3: Muguet accord:** 0.5% DPG of the Muguet accord A was replaced with 0.5% of select compounds (10, 9, and 6) and the different corresponding accords were applied at 0.6% in a shampoo base and at 1% in concentrated fabric softener base.

**Table 3: Example 3 - Muguet accords dosed with Compounds 10, 9, or 6.**

| | Accord 3A | Inventive Accord 3B | Inventive Accord 3C | Inventive Accord 3D |
|---|---|---|---|---|
| PHENYLETHYL ALCOHOL | 320 | 320 | 320 | 320 |
| HEDIONE^{™} | 195 | 195 | 195 | 195 |
| FLOROL^{™} | 130 | 130 | 130 | 130 |
| PHENOXANOL | 78 | 78 | 78 | 78 |
| PHENYLACETIC ALD. GLYCEROACETAL | 41 | 41 | 41 | 41 |
| MUGANE^{™} | 40 | 40 | 40 | 40 |
| HEXENOL CIS 3 | 36 | 36 | 36 | 36 |
| INDOL 10% DPG | 35 | 35 | 35 | 35 |
| BETAHYDRANE^{™} 10% DPG | 10 | 10 | 10 | 10 |
| MAYOL^{™} | 8 | 8 | 8 | 8 |
| ROSYRANE^{™} 10% DPG | 5 | 5 | 5 | 5 |
| VERBETRYLE^{™} 10% DPG | 2 | 2 | 2 | 2 |
| DIPROPYLENE GLYCOL - DPG | 100 | 95 | 95 | 95 |
| Compound 10 | - | 5 | - | - |
| Compound 9 | - | - | 5 | - |
| Compound 6 | - | - | - | 5 |

**Shampoo:** Accord 3A is a classical Muguet accord: floral, green rosy, quite representative of the lily of the valley flower. Inventive accord 3B (compound 10) brings strength to the accord, the note is more natural, green, even if less floral. Inventive accord 3C (compound 9) brings strength and a nice floralcy to the accord, the note is more lily of the valley, less rosy and technical. Inventive accord 3D (compound 6) brings a lot of strength, it could be less dosed. It changes the muguet profile by bringing a real marine tonality with more ozonic facets. This complexity is interesting giving a "à la Eau d'Issey (Issey Miyake)" note to the accord.

**Softener:** Accord 3A is greener, and the rosy note is more present, rendering the scent more technical. Inventive accord 3B (compound 10) gives strength to the accord and wraps up the green note to offer a green-muguet fragrance that is covering better the base. Inventive accord 3C (compound 9) enhances the floralcy of the accord, which is much more lily of the valley and with much efficacy on covering the base. Inventive accord 3D (compound 6) imparts another dimension to the accord by delivering marine and ozonic tonalities. Inventive accord 3D is more complex, less rosy, more like a fresh green-lily of the valley, with a clean aldehydic undertone.

**Example 4: Jasmine accord:** 0.5% DPG of the Jasmine accord A was replaced with 0.5% of select compounds (10, 9, and 6) and the different corresponding accords were applied at 0.6% in a shampoo base and at 5% in alcohol.

**Table 4: Example 4 - Jasmine accords dosed with Compounds 10, 9, or 6.**

| | Accord 4A | Inventive Accord 4B | Inventive Accord 4C | Inventive Accord 4D |
|---|---|---|---|---|
| BENZYL BENZOATE | 255 | 255 | 255 | 255 |
| HEDIONE^{™} | 220 | 220 | 220 | 220 |
| BENZYL ACETATE | 170 | 170 | 170 | 170 |
| INDOL 10% DPG | 85 | 85 | 85 | 85 |
| LINALOOL | 70 | 70 | 70 | 70 |
| HEXENOL CIS 3 10% DPG | 45 | 45 | 45 | 45 |
| PARACRESYL ACETATE 10% DPG | 16 | 16 | 16 | 16 |
| CIS JASMONE | 15 | 15 | 15 | 15 |
| EUGENOL | 12 | 12 | 12 | 12 |
| GERANIUM BOURBON EO | 12 | 12 | 12 | 12 |
| DIPROPYLENE GLYCOL - DPG | 100 | 95 | 95 | 95 |
| Compound 10 | - | 5 | - | - |
| Compound 9 | - | - | 5 | - |
| Compound 6 | - | - | - | 5 |

**Shampoo:** Accord A is a simple figurative Jasmine scent, a bit green and fruity, like the delicate petals of the flower. Inventive accord 4B (compound 10) pushes the benzyl notes, adding more fruitiness (banana hints from the benzyl acetate), almost changing the scent into an ylang flower. It also gives more strength to this accord. Inventive accord 4C (compound 9) gives roundness to the floral scent, the accord X is more cosmetic, more floral; in comparison Jasmine accord 4A seems to be more technical. Inventive accord 4D (compound 6) imparts ozonic facets, while rounding the floral note. Inventive accord 4D is more solar changing into a subtle frangipani scent.

**Alcohol (ethanol):** Similar to evaluation in shampoo, Accord A is a simple figurative Jasmine scent. Inventive accord 4B (compound 10) adds fruitiness, the scent is greener, more ylang. The floral note is powerful. Inventive accord 4C (compound 9) imparts more floralcy and roundness to the scent. Inventive accord 4C is more powerful and pleasant. Inventive accord 4D (compound 6) changes the note to a more marine and ozonic fragrance, less animalic, more solar and very powerful.

While the invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention in its broader aspects is therefore not limited to the specific details, representative product and/or method and examples shown and described. The various features of exemplary embodiments described herein may be used in any combination. Accordingly, departures may be made from such details without departing from the scope of the general inventive concept.

## Claims

1. A compound having a general Formula (I): 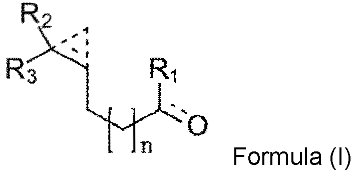
wherein n = 1 or 2;
wherein R¹ = H, methyl, ethyl, or alkoxy; and
wherein R² = H, methyl, or ethyl; and R³ = ethyl, 2,4-dimethylcyclohex-3-enyl, 1-ethylpropyl, 1,5-dimethylhex-4-enyl, cyclopentyl, 1,5-dimethylhexyl, 2,6-dimethylhepta-1,5-dienyl, cyclohexyl, 3,3-dimethylcyclohexyl, 2,6-dimethylhept-5-enyl, 2-phenylpropyl, 4-methylpent-3-enyl, hex-3-enyl, 1,5-dimethyl-5-methoxyhexyl, or 1-methylpropyl; or
wherein R² and R³ taken together form 4-propylcyclohexyl, 3,3-dimethylcyclohexyl, or 4-methylcyclohexyl.

2. The compound of claim 1, wherein the alkoxy is selected from C1-C5 alkoxy groups.

3. The compound of claim 1, selected from the group consisting of:
ethyl 6,10-dimethylundeca-4,9-dienoate; ethyl 6,10-dimethylundec-4-enoate; ethyl 3-(2-(6-methylheptan-2-yl)cyclopropyl)propanoate; 7,11-dimethyldodeca-4,10-dienal; 10-methoxy-6,10-dimethylundec-4-enal; 7,11-dimethyldodec-5-enal; ethyl 5-(4-propylcyclohexylidene)pentanoate; ethyl 6-(3,3-dimethylcyclohexyl)hex-5-enoate; 5-(3,3-dimethylcyclohexyl)hex-4-enal; and 6-ethyloct-4-enal.

4. A composition comprising one or more compounds according to any one of claims 1 to 3, said composition being in particular a fragrance composition, a flavor composition, a pharmaceutical composition, a cosmetic composition, a cleaning composition, a food composition, or an intermediate composition for the preparation of one of said compositions.

5. Use of at least one compound according to any one of claims 1 to 3 to confer, modify or enhance the organoleptic properties of a substance, composition, or article.
